# EUROPEAN PATENT APPLICATION

(11) **EP 1 693 459 A1**
(43) Date of publication of application: **23.08.2006**
(21) Application number: 04819350.2
(22) Date of filing: 24.11.2004
(51) Int. Cl.: C12N 15/63, C12N 7/01, A61K 35/76, A61K 48/00, A61P 31/18, A61P 37/04

(54) **CHIMERIC TYPE 5/TYPE 11 OR TYPE 35 ADENOVIRUS VECTOR FOR PREVENTING INFECTION WITH ANTIHUMAN IMMUNODEFICIENCY VIRUS**

(30) Priority: 28.11.2003 JP 2003399016
(71) Applicant: Aristo K.K., Kita-ku Kobe-shi, Hyogo 651-1513 (JP); Nichi-Iko Pharmaceutical Co. Ltd., Toyama-shi, Toyama 9300083 (JP)
(72) Inventor: SHIMADA, Masaru, Yokohama-shi, Kanagawa 2360005 (JP); OKUDA, Kenji, Yokohama-shi, Kanagawa 2350045 (JP)
(74) Representative: Becker Kurig Straus
(86) International application number: PCT/JP2004/017375
(87) International publication number: WO 2005/052165

(57) **Abstract**

A chimera adenovirus type 5/type 11 or type 35 vector which comprises a replication-defective adenovirus type 5 and a gene coding for a human immunodeficiency virus (HTV) envelope protein or for a mutant thereof equivalent in function thereto as integrated in the adenovirus type 5 in a manner capable of expression, with the fiber protein-encoding gene of said adenovirus type 5 being substituted by a gene coding for the fiber protein of an adenovirus type 11 or type 35 or for a mutant thereof equivalent in function thereto in a manner capable of expression shows reduced hepatotoxicity, can induce a very strong HIV-specific cellular immune response, and is very effective as a drug for the protection against HIV infection.

## Description

### Technical field

The present invention relates to a chimera adenovirus type 5/type 11 or type 35 vector for the protection against human immunodeficiency virus. In detail, present invention relates to a chimera adenovirus type 5/type 11 or type 35 vector which comprises a replication-defective adenovirus type 5 and a gene coding for a human immunodeficiency virus (HIV) envelope protein as integrated in said adenovirus type 5 in a manner capable of expression, with the fiber protein-encoding gene of said adenovirus type 5 being substituted by a gene coding for the fiber protein of an adenovirus type 11 or type 35 in a manner capable of expression and medical application thereof..

### Back ground of the invention

Highly active anti-retroviral therapy (HAART) has achieved great effect in the reduction of death rates of HIV-infected patients. However, there is a problem of a high incidence of adverse effects and drug-resistance in HAART, and the current situation in the developing countries is acquisition of therapeutic drugs for this disease is very difficult.
In addition, HAART does not exert an effect as a preventive drug against HIV infection, thus in future measures for AIDS control, the development of a vaccine with high safety and efficacy is becoming very important issue. Many literatures have reported that cytotoxic T lymphocyte (CTL) plays more important role than neutralization antibody in defense mechanism against HIV infection (nonpatent reference 1, nonpatent reference 2 and nonpatent reference 3). For the enhancement of cellular immunity, multiple strategies to produce an immunogenic HIV vaccine have been explored in the past decade. This included production of HIV subunit peptide vaccines, DNA vaccines, recombinant virus-vector vaccines including modified vaccinia Ankara (rMVA) (nonpatent reference 3 and nonpatent reference 4),adenovirus (nonpatent reference 5),rabies virus(nonpatent reference 6),flavivirus (nonpatent reference 7), freind mouse luekemia virus (nonpatent reference 8), Venezuelan equine encephalitis virus(nonpatent reference 9), vesicular stomatitis virus (nonpatent reference 10) adeno-associated virus( nonpatent reference 11 and (nonpatent reference 12), and bacterial vector vaccines (bacille Calmette.Guerin) (nonpatent reference 13) and Lactococcus (nonpatent reference 14.) Each of these strategies showed some promising results in animal models, either alone or in combination, and some of these have been reported to halt the progression of AIDS in experimental infection in non-human primates (nonpatent reference 3, nonpatent reference 4, nonpatent reference 5, nonpatent reference 8, nonpatent reference 15 and nonpatent reference 16). A conjugated vaccine comprising of DNA-priming followed by rMVA-boosting is one of the vaccination methods achieving great success and has now been conducted in phase I clinical studies.

Adenovirus type 5 (Ad5), which has been expected as a virus vector, is more immunogenic than rMVA (nonpatent reference 5) with an advantage of producing high virus titer and well analyzed information about its biological characteristics including DNA sequences. Ad5, however, has strong affinity to hepatic cells (nonpatent reference 17) and was reported that one patient with ornithine transcarbamylase (OTC) deficiency died by the treatment of Ad5 vector (non-patent reference 18), hence the use of this vector as clinical purpose has been limited.
Adenoviruses have 51 different serotypes and are classified into six subgroups consisting of A to F. Gene delivery system using an adenovirus vector currently adopts adenovirus type2 (Ad2) and adenovirus type 5(Ad5) (both belong to group C), because both Ad2 and Ad5 are well characterized and their DNA sequences are fully analyzed (nonpatent reference 19, nonpatent reference.20). Most of adenoviruses other than adenovirus type 3(Ad3), adenovirus type 11(Ad11), adenovirus type 35(Ad35) and adenovirus type 50(Ad50) (nonpatent reference 21, nonpatent reference.22)are known to bind coxsackievirus and adenovirus receptor (CAR) (nonpatent reference 7, nonpatent reference 9**,** nonpatent reference 13, nonpatent reference 15, nonpatent reference 22, nonpatent reference 23, nonpatent reference 24, nonpatent reference 25, nonpatent reference 26). CAR is expressed in heart, pancreas, central and peripheral nerve, prostate, testis, lung, liver and intestines of human, and especially, pretty amounts of CAR are expressed in liver. By this reason, Ad5 is considered to infect liver in human and animals easily.

It has been reported that a conjugated vaccine composed of DNA-priming followed by Ad5 vector-boosting demonstrate a remarkable protective effect against SHIV experimental infection in monkey (non-patent reference.27). However, because Ad5 has strong tropism especially to hepatic cells, hepatotoxicity is prone to occur. Due to this reason, safety as a virus vector vaccine is concerned.
nonpatent reference 1 Haynes, B. F. et al., Science 271:324-328,1996
nonpatent reference 2 Miedema, F. et al., Science 272:505-506,1996
nonpatent reference 3 Robinson, H. et al., Nat. Med. 5:526-534,1999
nonpatent reference 4 Amara, R. R. et al., Science 292:69-74,2001
nonpatent reference 5 Shiver, J. W. et al., Nature 415:331-335,2002
nonpatent reference 6 Schnell, M. J. et al., Proc. Natl. Acad. Sci. USA 9 7:3544-3549,200
nonpatent reference 7 Mandl, C. W. et al., Nat. Med. 4:1438-1440, 1998
nonpatent reference 8 Matano, T. et al., Vaccine 18:3310-3318,2000
nonpatent reference 9 Caley, I. J. et al., Vaccine 17:3124-3135,1999
nonpatent reference 10 Rose, N. F. et al., Cell 106:539-549,2001
nonpatent reference 1 1 Xin, K.-Q. et al., Hum. Gene Ther. 13:1571-1581, 2002
nonpatent reference 1 2 Xin, K.-Q. et al., Hum. Gene Ther. 12:1047-1061, 2001
nonpatent reference 1 3 Aldovini, A. et al., Nature 351:479-482,1991
nonpatent reference 1 4 Xin, K.-Q. et al., Blood 102:223-228,2003
nonpatent reference 15 Barouch, D. H. et al., J. Viol. 75:5151-5158,2001
nonpatent reference 1 6 Barouch, D. H. et al., Science 290:486-495,2000
nonpatent reference 17 Adams, J. Y et al., Nat. Med. 8:891-896,2002
nonpatent reference 1 8 Thomas, C. E. et al., Nat. Reviews Genetics 4:346-358,2003
nonpatent reference 19 Chroboczek, J. et al., Virol. 186:280-285,1992
nonpatent reference20 van Ormondt, H. et al., Curr. Top. Microbiol. Immunol. 110:73-142, 1984
nonpatent reference 21 Shayakhmetov, D. M. et al., J. Viol. 74:2567-2583,2000
nonpatent reference 22 Stevenson, S. C. et al., J. Viol. 69:2850-2857,1995
nonpatent reference 23 Amara, R. R. et al., J. Viol. 76:7625-7631,2002
nonpatent reference 24 Farina, S. F. et al., J. Virol 75:11603-13,2001
nonpatent reference 25 Jounai, N. et al., J. Gene Med. 5:609-617,2003
nonpatent reference 26 Lieber, A. et al., J. Viol. 70:8944-8960,1996
nonpatent reference 27 Lubeek, M.D. et al., Cell 106:539-549,1997

### Disclosure of the invention

### Assignment to be solved by the invention

Assignment of present invention is to provide a new virus vector using Ad5 which is usable for HIV vaccine with higher safety and efficacy. In addition, the assignment is to provide a pharmaceutical composition comprising said virus vector as an active ingredient, which is applied for the protection against HIV infection, HIV vaccine and HIV conjugated vaccine. And the assignment is to provide methods of prevention against HIV infection or HIV vaccine utilizing said virus vector.

### Measures to solve the assignment

The inventors keenly investigated aiming at the development of HIV vaccine with higher safety and efficacy. Thus, we constructed a chimera adenovirus type5 with Ad35 fiber expressing HIV genes. When antigen specific immune responses evoked by this chimera vector were assessed in BALB/c mice, it became obvious that strong HIV-specific cellular immune response was enhanced by intramuscular administration of the chimera vector, which were much higher when compared with enhancement elicited by an administration of the same titer of a recombinant vaccinia virus vector (Ankara strain) or a replication competent vaccinia virus vector (WR strain). Furthermore, immunization comprising of DNA-vaccine priming followed by this chimera vector-boosting demonstrated the complete protection against the infection of vaccinia virus expressing HIV genes at seven weeks after the boost immunization. This evidence suggests that the conjugated vaccine comprising of DNA vaccine and this chimera vector can become a breakthrough vaccine which controls the spread of AIDS. Present invention was completed on the basis of these evidences.

Therefore, the present invention refers to a chimera adenovirus type 5/type 11 or type 35 vector which comprises a replication-defective adenovirus type 5 and a gene coding for a human immunodeficiency virus envelope protein or for a mutant thereof equivalent in function thereto as integrated in said adenovirus type 5 in a manner capable of expression, with the fiber protein-encoding gene of said adenovirus type 5 being substituted by a gene coding for the fiber protein of an adenovirus type 11 or type 35 or for a mutant thereof equivalent in function thereto in a manner capable of expression.
Furthermore, present invention refers to a pharmaceutical composition which comprises, as an active ingredient, a chimera adenovirus type 5/type 11 or type 35 vector as described above. This pharmaceutical composition can be used for the prevention against the infection of human immunodeficiency virus and further used for a vaccine against human immunodeficiency virus. This pharmaceutical composition can also be used as a conjugated vaccine together with a replication-defective virus vector or non-virus vector in which human immunodeficiency virus gene is integrated in a manner capable of expression. The pharmaceutical composition can also be used with anti-HIV agents.
Moreover, this invention refers to a method for the protection against HIV infection or for a vaccine against HIV, which consists of the administration of above chimera adenovirus type 5/type 11 or type 35 vector and replication-defective virus vector or non-virus vector comprising a gene coding for a HIV envelope protein or for a mutant thereof equivalent in function thereto as integrated in a manner capable of expression. Furthermore, this invention refers to a method for the protection against HIV infection or for a vaccine against HIV, which consists of the administration of above chimera adenovirus type 5/type 11 or type 35 vector and anti-HIV agents.

### Effect of the invention

A chimera adenovirus type 5/type 11 or type 35 vector provided by this invention can elicit strong antigen specific cellular immune responses. This response is stronger than the immune responses induced by MVA vector, which is considered to be leading candidate for HIV vaccine. In addition, a conjugated vaccine combined with a chimera adenovirus type 5/type 11 or type 35 vector of this invention and DNA vaccine can induce a long-term protective responses against the virus in mouse infection model. Therefore a chimera type 5/type 11 or type 35 adenovirus vector of this invention shows the potential of the development of an epoch-making HIV vaccine to control the spread of AIDS. In addition, a chimera adenovirus type 5/type 11 or type 35 vector of this invention, when used with anti-HIV agents administered in the course of highly active anti-retroviral therapy (HAART), can strongly exert the effect for the treatment and prevention against HIV infection. Moreover, a chimera adenovirus type 5/type 11 or type 35 vector of this invention has reduced toxicity to liver and enhanced affinity to dendritic cells, because a gene coding for fiber protein of adenovirus type 5 is substituted by fiber protein-encoding gene of adenovirus type 11 or type 35.,

### Best mode for carrying out the invention

A chimera adenovirus type 5/type 11 or type 35 vector of this invention comprises a replication defective adenovirus type 5 and a gene coding for a human immunodeficiency virus envelope protein or for a mutant thereof equivalent in function thereto in a manner capable of expression, with the fiber protein-encoding gene of said adenovirus type 5 being substituted by a gene coding for the fiber protein of adenovirus type 11 or adenovirus type 35 or a mutant thereof equivalent in function thereto in a manner capable of expression.
An adenovirus type 5 whose early gene E1 or early genes E1 and E3 are deleted is preferable to use to construct a chimera adenovirus type 5/type 11 or type 35 vector. Either HIV envelope protein of HIV 1 or HIV 2 can be used for an HIV envelope (env) protein to be expressed in adenovirus type 5.
Various types of the clade (subtype) in HIV type 1 are known to exist. An envelope protein either of clade A, clade B, clade C, clade D or clade E can be used. Gene sequences coding for an envelope protein and its amino acid sequences are known to public and are described in the following literatures: Gao, F., et al., J. Virol. 70(3), 1651-1667 (1996); Ratner, L., et al., Nature 313 (6000), 277-284 (1985); Jounai, N. et al., J. Gene Med., 5, 609-617 (2003); Kim, F.M., et al., J. Virol. 69(3), 1755-1761 (1995); Rodenburg, C.M., et al., AIDS Res. Hum. Retroviruses 17(2), 161-168 (2001); Gao, F., et al., J. Virol. 72(7), 5680-5698 (1998); Gao, F. et al., J. Virology 70(10), 7013-7029 (1996). And those sequences are also available from NCBI as genetic information numbers of No. U51190, K03455, U39362, AF286224, U88822, U51188.
In this invention, an envelope protein is not restricted by the gene coding for the envelope protein itself, but a gene coding for a mutant thereof having equivalent immunogenicity thereto is included.
As an example like this mutant, proteins whose one or several residues are deleted, substitute and/or inserted in the amino acid sequence of the envelope protein, with having an equivalent immunogenicity to the envelope protein, are included. Or, a gene coding for a protein having an equivalent immunogenisity to the envelope protein with hybridizing to a gene coding for an envelope protein in the stringent condition, can also be included. A gene which are able to hybridize at 65°C for 12 hours in the solution containing 6 x SSPE, 2 x Denhart's solution, 0.5%SDS, 0.1mg/ml denatured salmon sperm DNA in the Southern Blot hybridization can be exemplified as a gene hybridizing in the stringent condition.
In this invention, a gene coding for an envelope protein of HIV clade B, HIV clade C or for a mutant thereof with equivalent function thereto is preferable. Furthermore, this invention covers a chimera adenovirus type 5/11 or type 35 vector integrated by a gene coding for HIV gag or for a mutant thereof which is concurrently integrated with a gene coding for envelope protein or for a mutant thereof. Gag gene codes for core protein of HIV, and its DNA sequences and deduced amino acid sequences are well known to the public. They are described in the following literatures: Gao, F., et al., J. Virol. 70(3), 1651-1667 (1996); Ratner, L., et al., Nature 313 (6000), 277-284 (1985); Jounai, N. et al., J. Gene Med., 5, 609-617 (2003); Kim, F.M., et al., J. Virol. 69(3), 1755-1761 (1995); Rodenburg, C.M., et al., AIDS Res. Hum. Retroviruses 17(2), 161-168 (2001); Gao, F., et al., J. Virol. 72(7), 5680-5698 (1998); Gao, F. et al., J. Virology 70(10), 7013-7029 (1996). Those sequences are also available from GENEBANK of NCBI as genetic information numbers of No. U51190, K03455, U39362, AF286224, U88822, U51188.
A mutant of the gag gene could also be included. A mutant gene described herein is defined as a gene coding for a protein having an equivalent function to the protein coded by the gag gene. As described in the part of the envelope protein above, a mutant gene is defined as a gene coding for a protein whose one or several residues are deleted, substituted or/and inserted in the amino acid sequence coded by the gag gene, with having equivalent function to the protein encoded by the gag gene, or defined as a gene coding for a protein having an equivalent function to the protein coded by the gag gene, with hybridizing to the gag gene under the stringent condition

A gene coding for envelope protein or for a mutant thereof, or a gene coding for gag protein or for a mutant thereof can be synthesized by PCR or other methods based on widely known genetic information. These syntheses, for example, according to the textbook of Molecular Cloning 2nd Edt., Cold Spring Harbor Laboratory Press (1989) and like other textbooks, can be easily conducted by those in the art. Furthermore, a gene coding for a mutant described above or a gene hybridized under the stringent condition, for example, can be easily prepared by the site directed mutagenesis method, common hybridization methods and other relevant methods, and more concretely, the preparation can be performed referring to textbooks like Molecular Cloning described above and others..
In this invention, rev gene, a regulatory gene of HIV, could be used with a gene coding for an envelope protein or for a mutant thereof. Information on rev gene is available from the following literatures: Gao, F., et al., J. Virol. 70(3), 1651-1667 (1996); Ratner, L., et al., Nature 313 (6000), 277-284 (1985); Jounai, N. et al., J. Gene Med., 5, 609-617 (2003); Kim, F.M., et al., J. Virol. 69(3), 1755-1761 (1995); Rodenburg, C.M., et al., AIDS Res. Hum. Retroviruses 17(2), 161-168 (2001); Gao, F., et al., J. Virol. 72(7), 5680-5698 (1998); Gao, F. et al., J. Virology 70(10), 7013-7029 (1996); Genetic information from GENEBANK, NCBI No. U51190, K03455, U39362, AF286224, U88822, U51188. To integrate a gene coding for HIV envelope protein or for a mutant thereof and a gene coding for gag gene or for a mutant thereof, together with rev gene according to need, into a replication-defective adenovirus type 5 in a manner capable of expression, gag gene or mutant thereof, rev gene and env gene or a mutant thereof are placed downstream of a CMV promoter(cytomegalovirus promoter) or CAG promoter(chimera promoter of CMV promoter and chicken β-actin promoter), for example, and genome of adenovirus type 5 is connected to both end of the expression unit composed of poly A sequences, and then are transfected into 293 cells derived from human embryonic kidney cell by the method of homologous recombination.

A gene sequences coding for an envelope protein of adenovirus type 11 or type 35 and its amino acid sequences utilized in this invention are already known to the public and are described in the following literatures: Mei, YF. et al., Virology 206 (1), 686-689, 1995; Dmitry M. et al., J. Virology, Mar. 2000, vol.74, 2567-2583; NCBI U10272; NCBI AAA66361.1; Stone, D. et al., Virology, 2003, Vol.309, 152-156. Fiber protein is made up by shaft and knob, and genes coding for fiber proteins composed by these are usually utilized for this invention.
In present invention, a gene coding for a fiber protein is not restricted by itself, but a gene coding for a mutant thereof having equivalent adhesion properties as possessed by the fiber protein, could be included. A mutant of the fiber protein described herein is also defined as a mutant thereof, whose one or several amino acid residues are deleted, substituted or/and inserted, having equivalent adhesion properties to the target cell as possessed by the fiber protein. This fiber protein also covers a gene coding for a mutant of the fiber protein whose adhesion properties are equivalent as that of the fiber protein, with hybridizing to a gene coding for the fiber protein under the stringent condition. Stringent condition, for example, includes the same condition as described above in the part of HIV envelope.protein. A gene encoding a fiber protein or its mutant can be easily synthesized by the widely known methods as is the case with a gene encoding a envelope protein or its mutant.
To replace a gene coding for fiber protein of a replication-defective adenovirus type 5 by a gene coding for adenovirus type 11 fiber protein or type 35 fiber protein or for a mutant thereof and integrate them into a replication-defective adenovirus type 5 in a manner capable of expression, adenovirus genes other than fiber are connected with both end of gene coding for adenovirus type 11 or type 35 or for a mutant thereof, and are transfected into 293 cells derived from human embryonic kidney cells, and thus a chimera adenovirus type 5/11 or type 5/35 vector can be constructed.

A chimera adenovirus type 5/11 or type 5/35 vector of this invention can also be easily constructed by use of a commercially available kit as shown below.
More specifically, for example, a chimera adenovirus type 5/11 or type 5/35 vector of this invention, which rev gene and env gene of HIVIIIB are integrated into E1,E3-depletion,replication-defective adenovirus type 5 in a manner capable of expression, with the fiber protein-encoding gene of said adenovirus type 5 being substituted by a gene coding for the fiber protein of adenovirus type 35, is easily constructed by use of a kit from Avior Therapeutics, Inc(Seattle, WA). In detail, for example, a 5.2k bp SalI/PstI fragment containing CAG promoter-HIVIIIB rev/env -polyA was isolated from pCAGrev/env described in Jounai,N. et al., J. Gene Med. 5:609-617,2003.
A left hand shuttle plasmid (pLHSP) containing Ad5 positions 22-342, Ad5 3523-5790, Escherichia coli ori, and ampicillin-resistant gene, and a right hand chimera shuttle plasmid (pRHSP5/35) containing E1,E3-deletion, replication-defective adenovirus type 5 in which a gene coding for fiber protein of said adenovirus type 5 is substituted by a gene coding for adenovirus type 35 (Ad35 shaft and Ad35 knob) in a manner capable of expression are obtained from Avior Therapeutics, Inc (Seattle,WA) as a construction kit.

The 5.2k bp SalI/PstI fragment containing CAG promoter-HIVIIIB revlenv -polyA is subcloned into EcoRI site, one of the multi-cloning site, of pLHSP containing Ad5 positions 22-342, Ad5 3523-5790, Escherichia coli ori, and ampicillin-resistant gene to generate pLHSP-HIV shuttle plasmid. The pLHSP-HIV shuttle plasmid is linearized with PacI and transfected together with a chimera shuttle plasmid vector, pRHSPS/35, into HEK293 cells using calcium precipitation method.
A chimera adenovirus type 5/35-HIV (Ad5/F35-HIV), the goal of this invention, can be obtained by incubating on agarose plate for 7-14 days after the transfection. Using similar methods, other chimera adenovirus type 5/type 11 or type 35 vector, another goal of this invention, can be generated.

A chimera adenovirus type 5/type 11 or type 35 vector of this invention can elicit very strong HIV-specific cellular immune response. Furthermore, since a chimera type 5/type 11 or type 35 adenovirus vector of this invention is constructed with the fiber protein-encoding gene of adenovirus type 5 being substituted by a gene coding for the fiber protein of adenovirus type 11 or type 35. Improved tropism to dendritic cells with reduced hepatotoxicity is achieved..
Thus, a chimera adenovirus type 5/type 11 or type 35 vector provided by this invention is highly effective as a protective medicine against HIV infection and as a vaccine for HIV.
When a chimera adenovirus vector is used together with a conventional replication-defective virus vector or a non-virus vector encoding HIV env gene or a mutant thereof equivalent in function thereto as integrated in a manner capable of expression, much higher HIV- specific cellular immune response can be elicited. Thus the conjugated vaccine composed of concomitant use of a chimera adenovirus vector with a conventional replication-defective virus vector or a non-virus vector is very efficacious as HIV vaccine.
These conventional replication-defective virus vector cover a recombinant adenovirus type 2 or type 5, in which early E1 gene is substituted by an expression unit encoding HIV env gene, or a replication-defective vacinia virus vector, in which an expression gene cluster encoding HIV env gene is transduced.
Non-virus vectors includes a expression vector usually used for mammalian cells such as pCAGGS (Gene 108: 193-200, 1991)), pBK-CMV, pcDNA3.1 and pZeoSV (Invitron Inc., Stratagene Inc.), in which a gene cluster expressing HIV envelope protein is transduced..
Furthermore, a chimera adenovirus type 5/type 11 or type 35 vector of this invention can be used with anti-HIV agents utilized for highly active anti-retroviral therapy (HAART). Reverse transcriptase inhibitors and protease inhibitors are well known as anti-HIV agents. Reverse transcriptase inhibitors are agents to inhibit the reverse transcription in which the viral RNA is converted into a normal DNA in the helper T cell. Azidothymidine(AZT), Didanosine(ddl), Lamivudine(3CT), Nevirapine(NVP) and others agents are well known among various reverse transcriptase inhibitors.
Protease inhibitors block protease, an enzyme, which HIV needs in order to make new viruses using protein synthesized from DNA, which is reverse transcribed from HIV RNA. Indinavir(IDV), Saquinavir(SQV), Ritonavir(RTV), Nelfinavir(NFV) and others are well known among various protease inhibitors.

Regarding the administration method of a chimera adenovirus type 5/type 11 or type 35 vector to human, following methods can be considered.
More specifically, for example, after a chimera adenovirus type 5/type 11 or type 35 adenovirus vector of this invention is dissolved in an appropriate solvent such as buffering solution like PBS, saline or sterilized water, it is subjected to filter sterilization, if necessary. Then an injectable solution is prepared by filling it in the aseptic container, and it is administered to human. Injectable solution herein prepared could be added a commonly used carriers, or subjected to freeze-dried formulation.
A chimera adenovirus type 5/type 11 or type 35 vector provided by this invention could be injected intravenously, intramuscularly, intraperitoneally, subcutaneously or intracutaneously. It could also be administered intranasally or orally. The dosage of a chimera adenovirus type 5/type 11 or type 35 vector of this invention vary according to a subject to be administered, method of administration and mode of administration, but usually the range of 10⁹ - 10¹² viral particles, preferably, 10¹¹ - 10¹² viral particles per an adult is considered to be dosed.
When a chimera adenovirus type 5/type 11 or type 35 adenovirus vector of this invention is administered with replication-defective virus vector or non-virus vector as described above, or with anti-HIV agents, similar manner of administration described above could be adopted, and a replication-defective virus vector or non-virus vector, or anti-HIV agents can be administered in a manner of administration method and dosage which are usually adopted..
The following examples are offered to more fully illustrate the invention, but are not to be construed as limiting the scope thereof.

### Example 1

### (1) Construction of a chimera adenovirus type 5/type 35 vector of this invention

A chimera adenovirus type 5/type 35 vector of this invention, which rev gene and env gene of HIVIIIB from HIV clade B strain were integrated into E1,E3-depletion,replication-defective adenovirus type 5 in a manner capable of expression, with the fiber protein-encoding gene of said adenovirus type 5 being substituted by a gene coding for the fiber protein of adenovirus type 35, was constructed by use of a kit from Avior Therapeutics, Inc(Seattle, WA) as described below.
The 5.2k bp SalI/PstI fragment containing CAG promoter-HIVIIIB rev/env-polyA was isolated from pCAGrev/env described in Jounai, N. et.al., J. Gene Med. 5:609-617, 2003.
A left hand shuttle plasmid (pLHSP) containing Ad5 positions 22-342, Ad5 3523-5790, Escherichia coli ori, and ampicillin-resistant gene and a right hand chimera shuttle plasmid (pRHSP5/35) containing E1,E3-deletion, replication-defective adenovirus type 5 in which a gene coding for fiber protein of said adenovirus type 5 is substituted by a gene coding for adenovirus type 35 (Ad35 shaft and Ad35 knob) in a manner capable of expression were included in a construction kit purchased from Avior Therapeutics, Inc (Seattle,WA).

Firstly, The 5.2k bp SalI/PstI fragment containing CAG promoter-HIVIIIB rev/env-polyA was subcloned into EeoRI inulti-cloning site of pLHSP containing Ad5 positions 22-34-2, Ad5 3523-5790, Escherichia coli ori, and ampicillin-resistant gene to generate pLHSP-HIV shuttle plasmid. The pLHSP-HIV shuttle plasmid was then linearized with PacI and transfected with a chimera shuttle plasmid vector, pRHSP5/35, to HEK293 cells using calcium precipitation methods.
A plaque of the chimera adenovirus type 5/ type -HIV (Ad5/35-HIV) was obtained by cultivation on the plate dish for 7-14 days after the transfection. The chimera adenovirus type 5/35-HIV, the goal of this invention, was propagated in HEK293 cells and purified by two repetitions of the CsCl methods.

### (2) Other recombinant vectors

Other vectors used in subsequent experiments were obtained as follows:
A replication-defective vaccinia virus expressing HIV env gene (Ankara strain, MVA-HIV) (Amara, R. R. et al., Science 292:69-74,2001; Amara, R. R. et al., J. Viol. 76:7625-7631,2002; Robinson, H. et al., Nat. Med. 5:526-534,1999) was provided by Dr. Moss (Laboratory of Viral Diseases, National Institutes of Health, MD). A replication competent vaccinia virus vector (WR strain, vPE16) was purchased from AIDS Reagent Program, National Institutes of Health, MD (Cat. No. 362). These recombinant vacinia virus was propagated in primary chicken embryo fibroblast cells and CV1 cells, respectively.

A chimera adenovirus type 5/type 35 vector (Ad5/F35-LacZ) in which galactosidaseZ (LacZ) is integrated into E1,E3-depletion,replication-defective adenovirus type 5 in a manner capable of expression, with the fiber protein-encoding gene of said adenovirusaype 5 being substituted by a gene coding for the fiber protein of adenovirus type 35, and E1,E3-depletion,replication-defective adenovirus type 5 expressing galactosidaseZ (Ad5-LacZ) were previously reported (Mizuguchi, H. et al., Gene 285:69-77,2002).
The total concentration of virus virions in each preparation was calculated from the optical density at 260nm (OD₂₆₀), using formula 1 OD₂₆₀= 1x10¹² viral particles /ml=1x10¹⁰ plaque forming unit (PFU)/ml. DNA vaccine (pCAGrev/env) containing HIV_{IIIB} and env has been previously reported(Jounai, N. J. Gene Med., 5:609-617; 2003).

### (3) Expression of the recombinant virus

### 1) Method

In order to confirm the expression of recombinant viruses, solution of MVA-HIV, vPE16(HIV env expressing replication competent vaccinia virus WR strain) and Ad5/F35-HIV are dissolved in the same amount of 2 xSDS buffer(125 mM Tris-HCl, pH 6.8, 4% SDS, 20% glycerol, 0.01% broiriophenol blue, 10% β- mercaptoethanol). After boiling for 10 minutes, this solution was subjected to 4-12% density gradient acrylamide gel electrophoresis. After electrophoresis, proteins were transferred to Hybond ECL nitrocellulose membrane (Amersham Pharmacia Biotech, Buckinghamshire, England) and then gp 160 was added by mouse anti-HIV gp 120 monoclonal antibody (hybridoma 902, AIDS Research and Reference Reagent Program, National Institute of Health, MD). After adding affinity-purified horseradish peroxidase (HRP) labeled mouse Ig (ICN Pharmaceuticals, Inc, OH), coloured products were photographed by ECL Western Blotting Detection System (Amersham Pharmacia Biotech).

### 2) Results

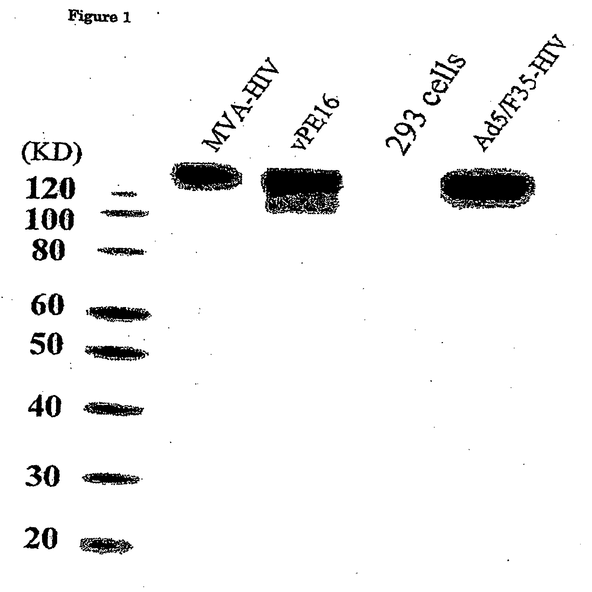

In vitro expression of MVA-HIV, vPE16 and Ad5/F35 were confirmed. Results were shown in figure 1. As shown in Figure 1, gp 160 was detected from lysates prepared from infected cells but not detected from non-infected 293 cells, thus the expression of HIV gene, as a consequence of infection of virus vector, was confirmed to express.

### Example 2

### Evaluation of recombinant virus

### (1) Method

### 1) Animals and immunization

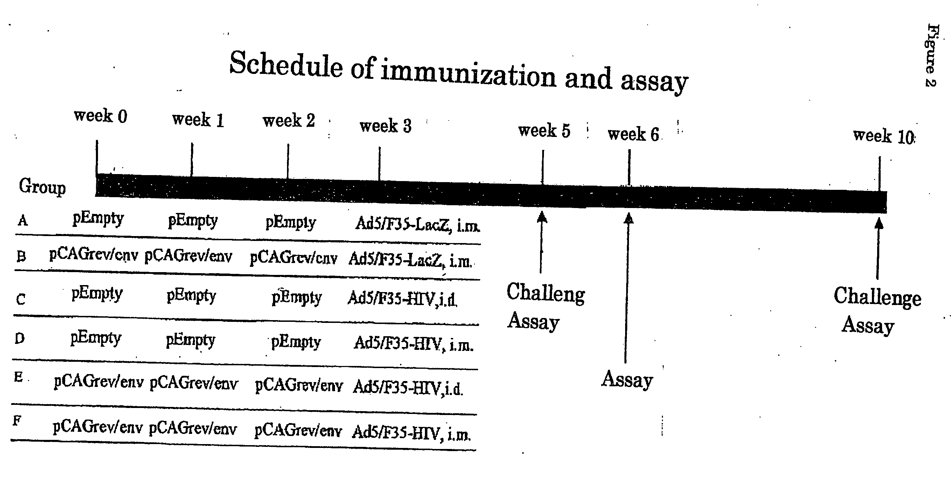

Eight-week-old female BALB/c mice were purchased from Japan SLC Inc., Shizuoka, Japan and kept in animal facility center with 12hr day-night rotating shift. Immunization methods are shown in Figure 2. The mice were immunized an i.m injection of 100 µg of pCAGrev/env or pCAGempty plasmid(rev/env deleted plasmid) DNA in phosphate-buffered saline (PBS) at 0,1, and 2 weeks and were boosted with an i.m. injection of 10¹⁰vp of Ad5/F35-LacZ or i.m. or i.d. injection of 10¹⁰vp of Ad5/F35-HIV. For groups of mice immunized with virus vector alone, 10¹⁰vp of Ad5/F35-Lacz or Ad5/F35-HIV in PBS were dosed with i.m., i.p., s.c., or i.d injection.

### 2) Tetramer assay

The tetramer assay was conducted one week after final immunization. PE-conjugated H-2Dd/p18 tetramer (RGPGRAFVTI) was purchased from AIDS Research and Reference Reagent Program, National Institute of Health, MD.
The tetramer assay was performed as described in Xin, K.-Q. et al., Hum. Gene Ther. 13:1571-1581,2002. Isolated lymphocytes were blocked for 30min at 4°C with 4% normal serum in PBS and stained with FITC-conjugated anti-mouse CD8 antibody(0.5µg/10⁶ cells) (Ly-2, PharMingen) for 30 min at 4°C. After washing twice with the staining buffer (3% FCS, 0.1% NaN₃ in PBS), the cells were incubated with the tetramer reagent for 15 min at 37°C, followed by flow cytomeric analysis (Becton Dickinson).

### 3) Intracellular cytokine staining assay

IFN γ -secreting CD8+ T cells were detected by the protocol recommended by the manufacturer (Cytofix/ CytoPerm Plus kit, PharMingen, San Diego, CA, USA). Lymphocytes were isolated from the mouse spleen. A single cell suspension was incubated with 10 µg/ml of the HIV V3 peptide (NNTRKRIQRGPGRAFVTIGKIGN) for 24 h at 37°C. At 2 h before the end of incubation, 1 µg/ml of GolgiPlug was added. The cells were washed with staining buffer (3% fetal calf serum (FCS), 0.1% sodium azide (NaN3) in PBS), blocked with 4% normal mouse sera, and stained with phycoerythrin (PE)-conjugated anti-mouse CD8 antibody (Ly-2, PharMingen). The cells were then suspended in 250 µl of Cytofix/Cytoperm solution at 4°C for 20 min, washed with Perm/Wash solution, and stained with anti-mouse IFN-g Ab conjugated with FITC (PharMingen) at 4°C for 30 min, followed by flow cytometric analysis.

### 4) Recombinant vaccinia virus used for the challenge study

The virus challenge experiment was carried out according to methods described in Belyakov, I. M. et al., Proc. Natl. Acad. Sci. USA. 95:1709-1714,1998 and Xin, K.-Q. et al., Hum. Gene Ther. 13:1571-1581,2002. Recombinant vaccinia virus,vPE16 (AIDS Reagent Program, NIH, MD; Cat. No. 362), expressing HIV-IIIB gene was used for the infection. Firstly, the mice were intraperitoneally challenged with 10⁸ PFU of vPE16 at 2 or 7 weeks after the immunization of chimera adenovirus vector Ad5/F35-HIV of this invention. At 6 days after challenge, the mice were killed, their ovaries were sonicated, and the vPE16 titer was determined by serial 10-fold dilution on a plate of CV1 cells. Infected cells were detected by staining with crystal violet and plaques were counted at each dilution.

### 5) Data analysis

All values were expressed as means ± standard error (S.E.). Data were conducted with one-way factorial analysis of variance. Significance was defined at P < 0.05 in the statistical analysis.

### (2) Results

### 1) Toxicity of adenovirus vector on liver and kidney cell.

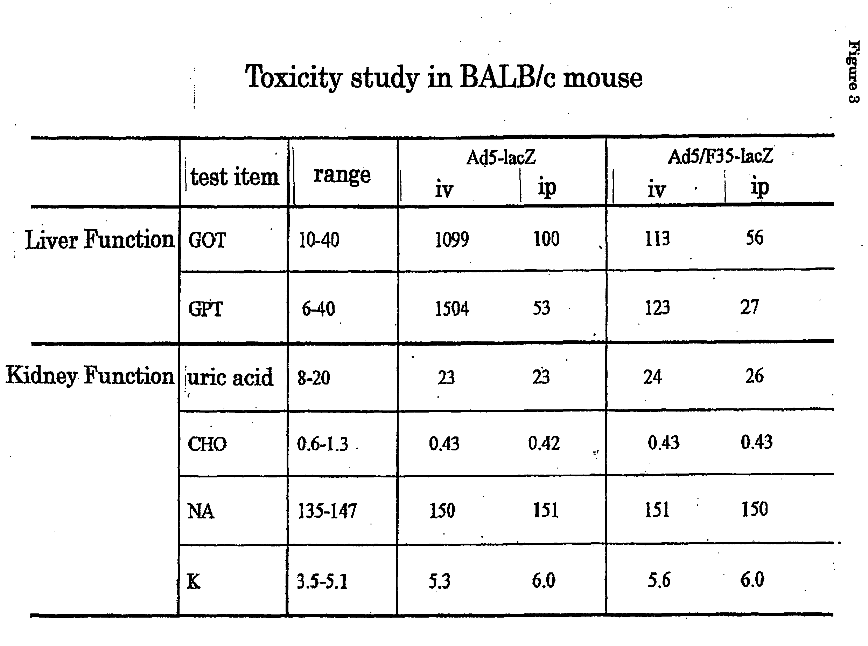

The mice were received an i.v. or i.p injection of 10¹¹ particles (10-fold higher amounts than immunization dosage) of Ad5-LacZ and Ad5/F35-LacZ. The mice were transferred to Kitayama Rabes Corp., Nagano, Japan, on days 6 after administration, where the toxicity studies on liver and kidney was conducted. As shown in Figure 3, Administration group of Ad5-LacZ showed 5-fold and 2-fold higher GOT and GPT value than Ad5/F35-HIY, respectively. Big difference of toxicity between group of Ad5-LacZ and Ad5/35-LacZ on kidney cells was not observed.

### 2) Time-course study of immune responses

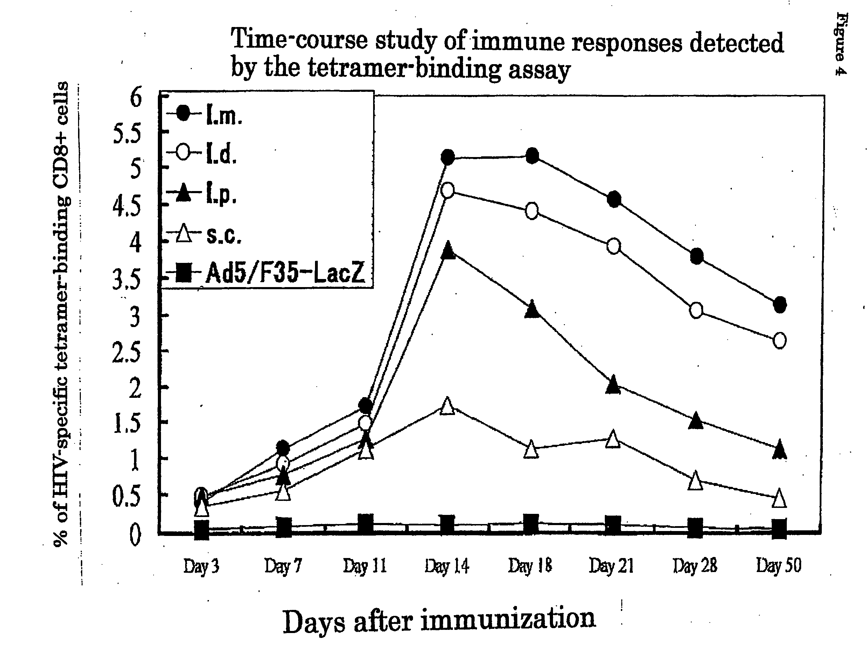

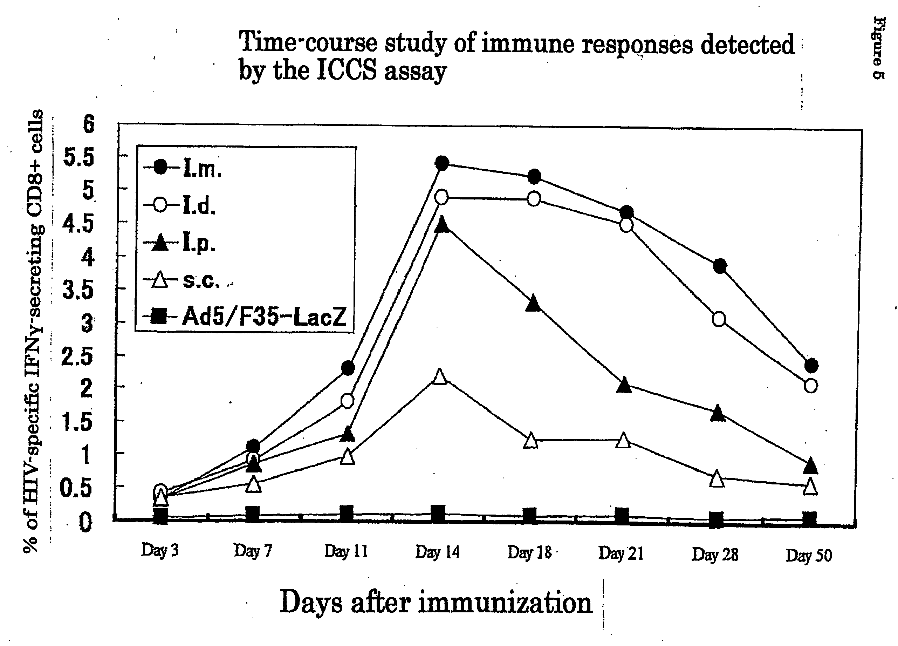

The mice were immunized with an i.m., i.d., or s.c. injection of Ad5/F35-HIV (10¹⁰ particles/mouse) and with an i.m. of Ad5/F35-LacZ (10¹⁰ particles/mouse) as negative control at day 0. HIV-specific immune responses of immunized mice were detected by the tetramer-binding assay and the intracellular cytokine staining assay (ICCS). As shown in Figure 4, the maximum tetramer-binding activity was observed on day 14 after immunization, and its activity decreased gradually after that. The tetramer-binding score in Figure 4 became greater in the order of i.m. > i.d. >i.p. >s.c., and reflect the strength of HIV-specific cell-mediated immune responses. The similar results were observed on ICCS assay(Figure 5). The background value (Ad5/F35-LacZ group) was less than 0.15% for tetramer-binding assay and less than 0.13% for ICCS assay. From these study results, i.m. and i.d. administration were selected for subsequent studies.

### 3) Long-term cell-medicated immune responses elicited by immunization of pCAGrev/env/Ad5/F35-HIV

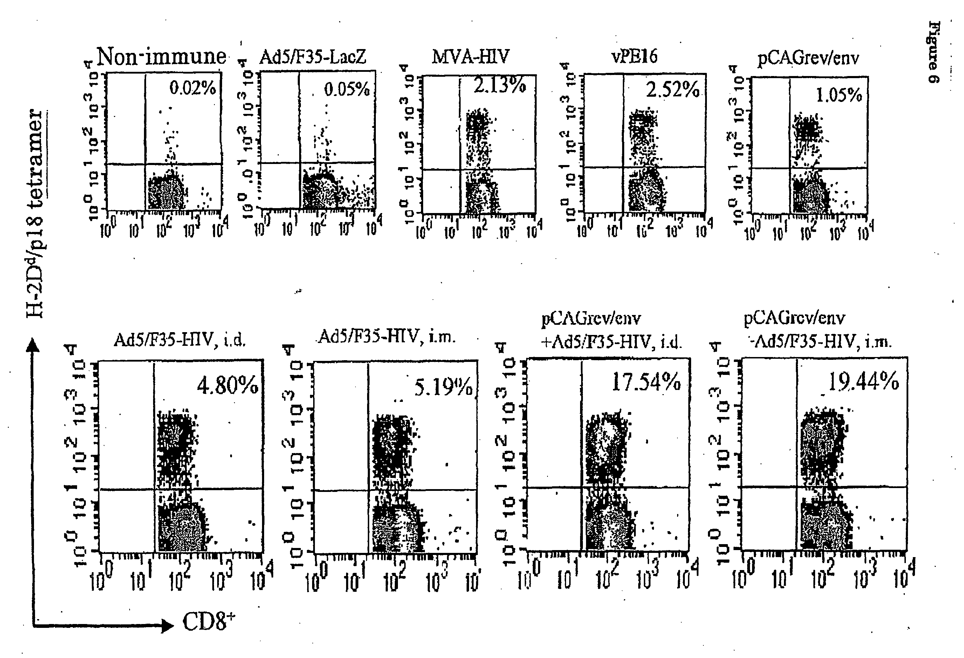

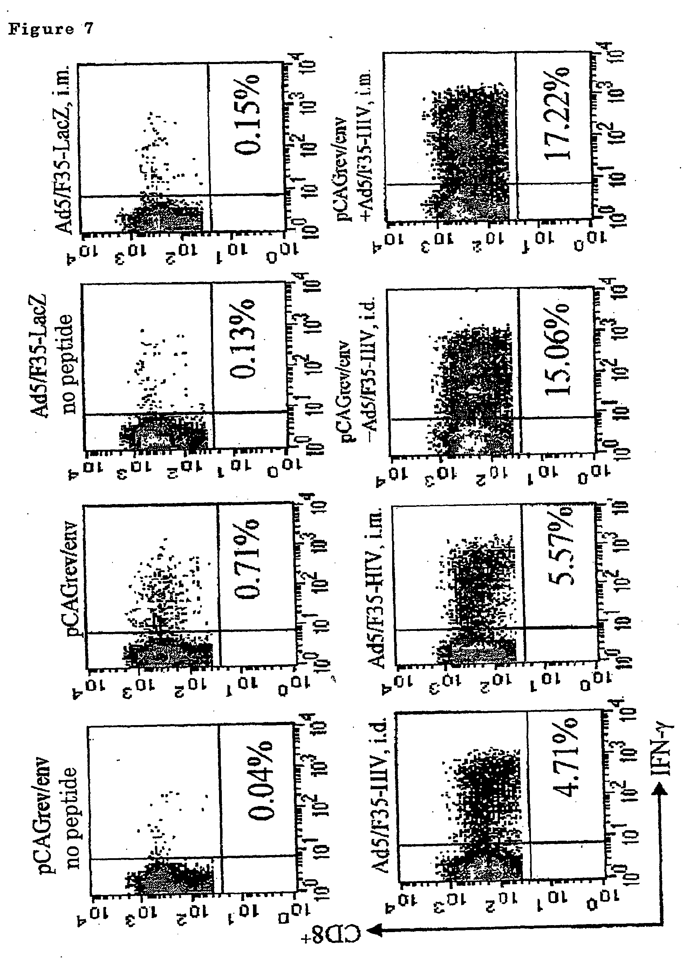

Two methods (tetramer-binding assay and intracellular cytokine staining assay) were performed to detect HIV-specific cell-mediated immune responses. Administration group of an i.m. injection of Ad5/F35-BW and an i.d. injection of Ad5/F35-HIV induced more than six-fold higher HIV-specific IFNγ-secreting CD8+ T cells than the group of DNA vaccine (pCAGrev/env) at 2 weeks after the final immunization (Figure 6). And Ad5/F35-HIV elicited about two-fold stronger cellular immunity than MVA-HIV, which is considered as leading candidate HIV vaccine, and vPE16. Furthermore, the conjugated vaccine composed of pCAGrev/env and Ad5/F35-HIV enhanced more than three-fold stronger immune responses than Ad5/F35-HIV alone. The similar results were also observed in ICCS assay (Figure 7). And when the mice immunized with pCAG/rev/env/Ad5/F35-HIV were subjected to booster immunization, HIV-specific immune response did not increase much (only increase from 18% to 20% for i.d. administration and from 19% to 21% for i.p. administration). From these observations, it was suggested that almost the highest antigen specific immune responses was elicited by the vaccination.

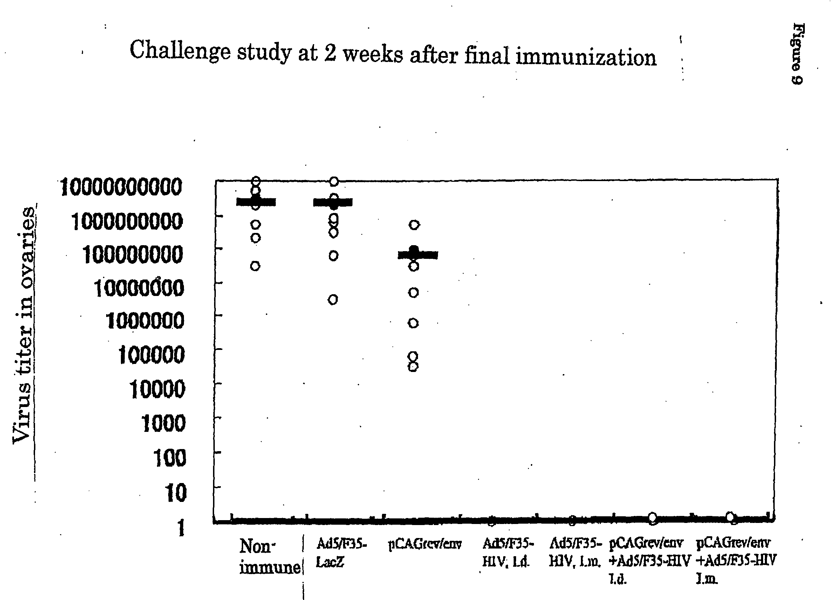

In order to determine if the immune responses induced by Ad5/35-HIV administration could contribute to the protection against infection, vaccinated mice were challenged intraperitoneally with vPE 16 expressing HIV env gene 2 weeks after final immunization. As shown in Figure 9**,** both group of Ad5/F35-HIV and pCAGrev/env/Ad5/F35-HIV protected mice completely against virus infection, thus it became obvious that these vaccines could contribute to host defense system..
Next, investigation was conducted to see if these vaccines could generate and maintain long term immune responses. Group of mice dosed pCAGrev/envAdS/F35-HIV vaccine was confirmed to maintain high level of HIV specific cell-mediated immune responses even 7 weeks after final immunization (8% and 9% for i.d. and i.m. administration, respectively) (Figure 10). The challenge study of vPE16 carried out 7 weeks after final immunization revealed that complete protection against virus infection was achieved by administration of pCAGrev/env/Ad5/F-35-HIV vaccine (Figure 11). Thus, it became obvious that this vaccine could induce long-term immune response. And Ad5/F35-HIV of this invention had 1000-fold higher virus titer than MVA-HIV as shown in Figure 12.

### Example 3

### (1) Construction of a chimera Adenovirus type 5/type 35 vector containing HIV clade C Env gene/ Gag gene

A chimera adenovirus type 5/35 vector of this invention, which env gene (gp 120, 2kbp) and gag gene (1.5kbp) of HIV clade C (96ZM651.8 strain) are integrated into E1,E3-depletion,replication-defective adenovirus type 5 in a manner capable of expression, with the fiber protein-encoding gene of said adenovirus type 5 being substituted by a gene coding for the fiber protein of adenovirus type 35, was constructed by homologous recombination method as described below.
Entire HIV clade C gp 120 encoding gene (2kbp, C/gp120) and gag-encoding gene (1.5kbp, C/gag) (GeneBank No. AF286224) were amplified in recombinant vaccinia virus vT331 strain (Gao F. et al., J. Virol., 1998, Vol. 72, 5680-5690). DNA fragment of C/gp120 and C/gag were synthesized by PCR using following primer:
- C/gp120-5'primer:: aagaattcctcgagaaaatgagagtgagggagatact
- C/gp120-3'primer:: aatctagatttttctctctccaccactctcc
- C/gag-5'primer:: aagtcgacaaaatgggtgcgagagcgtcaatat
- C/gag-3'primer:: aatctagattgagacaaggggtcgctgccaaa
Obtained C/gp 120 and C/gag fragments were fused with IgG Fc fragment (Barouch, D.H. et al., Proc. Natl. Acad. Sci. USA, 97, 4192-4197 (2000)) to generate C/gp120-Ig and C/gag-Ig. C/gp 120-Ig and C/gag-Ig was subcloned into XhoI and SalI site of pIRES plasmid (Cloneteck Inc.) to generate C/gp120-Ig-IRES-C/gag-Ig. TheC/gp120-Ig -IRES-C/gag-Ig fragment was subcloned into XhoI site of pCAGGS plasmid (Jounai, N. et al., J. Gene Med., 5, 609-617 (2003)). CAG promoter-C/gp120-Ig -IRES-C/gag-Ig-polyA fragment was subcloned into EcoRI multi-cloning site of LHSP vector, purchased from Avior Therapeutics Inc., to generate pLHSP-HIV clade C shuttle vector plasmid. pLHSP-HIV clade C shuttle plasmid and pRHSP shuttle vector were transfected into HEK 293 cells with lipofection reagent (Lipofectamine 2000). Virus Plaque was obtained from cultivation on agarose plate for 7-10 days after transfection. The recombinant viruses were propagated in HEK293 cells and purified by two repetitions of the CsCl methods. Ad5/35 -HIV clade C /env/gag vector carrying HIV clade C was thus isolated.

### (2) IFN-γ ELISpot assay

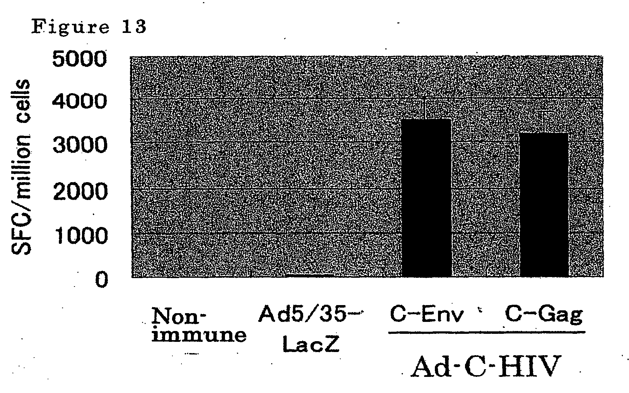

Mouse IFN-γ-secreting T cells were assessed by ELISpot assay (Cat. 3321-2A-2, MabTech, Nacka, Sweden) one week after the final immunization according to the manufacture's instruction. MultiScreen-IP plates (Millipore, Bedford, MA) was washed with 70% ethanol and PBS. Plates were coated with 10 µg/ml of anti-mouse IFN-γ monoclonal antibody (An18) in PBS overnight at 4° C. The plate was then washed and blocked with RPMI 1640 medium containing 10% FCS for 2 h at room temperature. Lymphocytes isolated from spleen or lymph nodes (1-10× 10⁵) were added to each well. The lymphocytes were stimulated with 10 µg/ml of HIV clade C V3 peptide (NNTRQSIRIGPGQTFYATGDIIGD) and HIV clade C gag peptide (DIKQGPKESFRDYVDRFFKTLR) for 24 h at 37° C. Lymphocytes in wells without stimulant were used as negative control.
After incubation, the plate was washed and cells were incubated with 1 µg/ml of biotinylated anti-mouse IFN-γ antibody (R4-6A2), followed by adding alkaline phosphatase streptavidin and 50 µl/well of BCIP/NBT phosphatase substrate (Kirkegaare and Perry Laboratories, Gaithersburg, MD).
The numbers of spots were automatically determined by Comuter Video Image Analyzer (KS Elispot, Carl Zeiss, Co., Ltd., Tokyo, Japan). All experiments were conducted in triplicate.
The BALB/c mice were immunized with an i.m. injection of 10¹⁰ particles of Ad5/35-LacZ or Ad5/35-HIV clade C /env/gag. HIV-specific IFN-γ secreting spleen cells were detected 2 weeks after the immunization. Obtained results were shown in Figure 13.

### (3) Results

As shown results in Figure 13, only less than 50SPC/million IFN-γ secreting spleen cells were detected in both non-immunized mice and mice dosed Ad5/35-LacZ. On the other hand, the group of Ad5/35-HIV clade C /env/gag, which was stimulated either by env or gag peptide, was detected significant higher number of IFN-γ secreting spleen cells.

### Usability in the industry

It has been proven by this invention that a chimera adenovirus type 5/type 11 or type35 vector carrying HIV env gene elicits strong antigen specific cell-mediated immune responses. The immune responses induced by this invention are stronger than that induced by MVA vector, which is considered as leading HIV vaccine candidate. In addition, the conjugated vaccine composed of a chimera Ad5/F35-HIV vector and DNA vaccine showed to induce long-term protective immune response against virus infection in mice experimental model. These results indicate the probability of the development of HIV vaccine to control the spread of AIDS.
Antigen specific cell-mediated immunity and neutral antibody plays a very important role for the protection against HIV infection. It became obvious that almost highest cell mediated immunity can be induced against a specific antigen by adopting the combination of DNA vaccine and a chimera adenovirus type 5/11 or type 35 vector expressing HIV env gene provided by this invention. Furthermore, this conjugated vaccine was shown to induce long-term protective immune responses.
And, a chimera adenovirus type 5/type 11 or type 35 vector provided by this invention can be used with anti-HIV agents for highly active anti-retroviral therapy (HAART). A chimera adenovirus type 5/type 11 or type 35 vector provided by this invention has reduced toxicity on liver cells with high affinity to human dendritic cells. Hepatotoxicity is the most critical barrier to use conventional Ad5 vector for clinical therapy. From these advantageous points, it can be expected to show excellent efficacy in the clinical trials.
Safe, effective and low-priced HIV vaccine which can induce cellular and humoral immunity is very important to control the spread of HIV infection. Thus, a chimera adenovirus type 5/type 11 or type 35 vector provided by this invention can become the next generation vaccine with great potential to control the spread of AIDS.

### Brief description of the drawings

### [Figure 1]

Recombinant MUA-HIV, vPE16 and Ad5/F35-HIV were subjected to electrophoresis, then transferred to nitrocellulose membrane and stained by mouse anti-HIV gp120 monoclonal antibody. Figure 1 shows the confirmation of expression of HIV gp 160 protein.
[Figure 2]
   Figure 2 shows the immunization protocol of various recombinant viruses in mice.
[Figure 3]
   Figure 3 shows the toxicity study results of Ad5 vector (Ad5-LacZ) and Ad5/F35 vector (Ad5/F35-LaeZ). At seven days after BALB/c mice (n=3) were immunized with an i.v or i.p. injection of 10¹¹ particles of Ad5 vector (Ad5-LacZ) or Ad5/F35 vector (Ad5/F35-LacZ), which was ten-fold higher viral particles than the dosage used in immunization, analyses on mice serum were conducted to determine toxicity to liver and kidney cell.
[Figure 4]
   Figure 4 indicates time-course study of HIV-specific immune response detected by the tetramer-binding assay when mice were immunized with a recombinant Ad5/F35-HIV The mice were dosed 10¹⁰ viral particles of Ad5/F35-HIV at day 0 via various administration routes. Ratio (%) of HIV specific CD8 cells were detected at the time point as indicated in the figure. Each plot was mean value calculated from 4-5 mice. In the Figure 4, i.m., i.d., i.p., and s.c. indicate intramuscular, intradermal, intraperitoneal, and subcutaneous administration, respectively. Ad5/F35-LacZ was Ad5/F35 vector expressing LacZ protein and thus used as negative control.
[Figure 5]
   Figure 5 indicates time-course study of HIV-specific immune response detected by the ICCS assay when mice were immunized with a recombinant Ad5/F35-HIV.
   The mice were dosed 10¹⁰ viral particles of Ad5/F35-HIV at day 0 via various administration routes. Ratio (%) of HIV-specific CD8 cells were detected at the time point as indicated in the figure. Each plot was mean value calculated from 4-5 mice. In the Figure 5, i.m., i.d., i.p., and s.c. indicate intramuscular, intradermal, intraperitoneal, and subcutaneous administration, respectively. Ad5/F35-LacZ was Ad5/F35 vector expressing LacZ protein and thus used as negative control.
[Figure 6]
   Figure 6 shows immune response after the final immunization of Ad5/F35-HIV of this invention. The mice were immunized with an i.m., or i.d. injection of DNA vaccine (pCAGrev/env) and/or virus vector (Ad5/F35-HIV) (10¹⁰ viral particles/mouse), either alone or in combination. HIV-specific immune response was detected by the tetramer-binding assay at 2 weeks after final immunization.
[Figure 7]
   Figure 7 shows immune response after the final immunization of Ad5/F35-HIV of this invention. The mice were immunized with an i.m., or i.d. injection of DNA Vaccine (pCAGrev/env) or virus vector (Ad5/F35-FHV) (10¹⁰ viral particles/mouse), either alone or in combination. HIV-specific immune response was detected by ICCS assay at 2 weeks after final immunization.
[Figure 8]

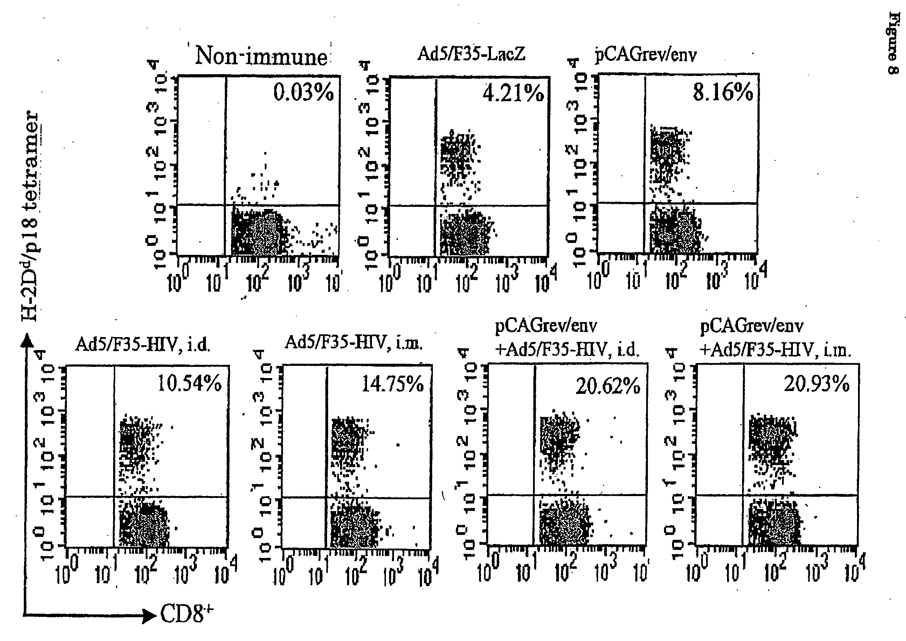

   Figure 8 shows immune response after the final immunization of Ad5/F35-HIV of this invention followed by the challenge of virus. The mice were immunized with an i.m., or i.d. injection of DNA vaccine (pCAGrev/env) or virus vector (Ad5/F35-HIV) (10¹⁰ viral particles/mouse), either alone or in combination. Immunization was conducted according to the schedule shown in Figure 2.The mice were challenged by an i.p. injection of 10⁸ pfu/mouse of vaccinia virus vPE16 at 2 weeks after the final immunization (week 5). Figure 8 shows the immune response one week after the challenge (week 6).
[Figure 9]
   Figure 9 shows immune response after the final immunization of Ad5/F35-HIV of this invention followed by the challenge of virus. The mice were immunized with an i.m., or i.d. injection of DNA vaccine (pCAGrev/env) or virus vector (Ad5/F35-HIV) (10¹⁰ viral particles/mouse), either alone or in combination. Immunization was conducted according to the schedule shown in Figure 2. The mice were challenged by an i.p. injection of 10⁸ pfu/mouse of vaccinia virus vPE16 at 2 weeks after the final immunization (week 5). Figure 9 shows the vPE16 titer in mouse ovaries at 1 week after the challenge (week 6). Each spot represents the virus titer of each mouse. A bar in the figure represents mean values of virus titers of 10 mice.

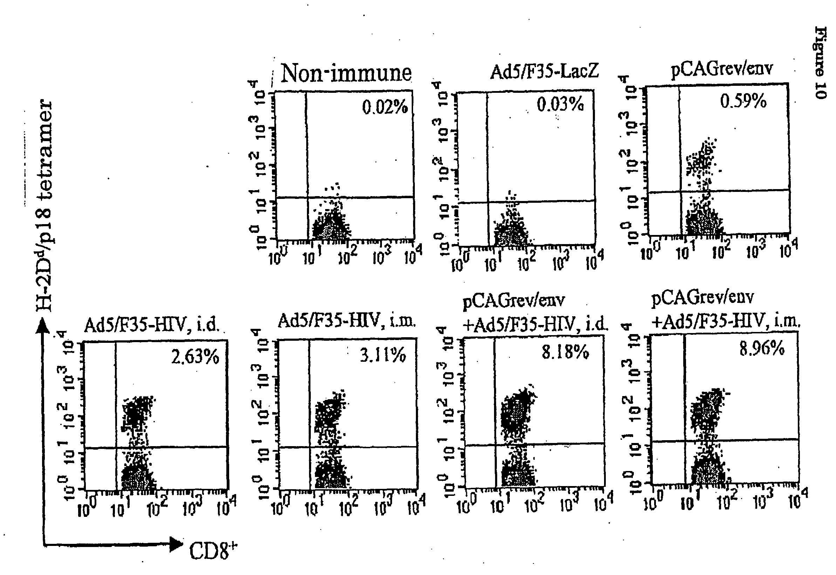

[Figure 10] Figure 10 shows immune response and the infection experiments results at 7 weeks after the final immunization. The mice were immunized with an i.m., or i.d. injection of DNA vaccine (pCAGrev/env) or virus vector (Ad5/F35-HIV) (10¹⁰ viral particles/mouse), either alone or in combination. Figure 10 shows HIV-specific immune response detected by tetramer-binding assay at 7 weeks after the final immunization.

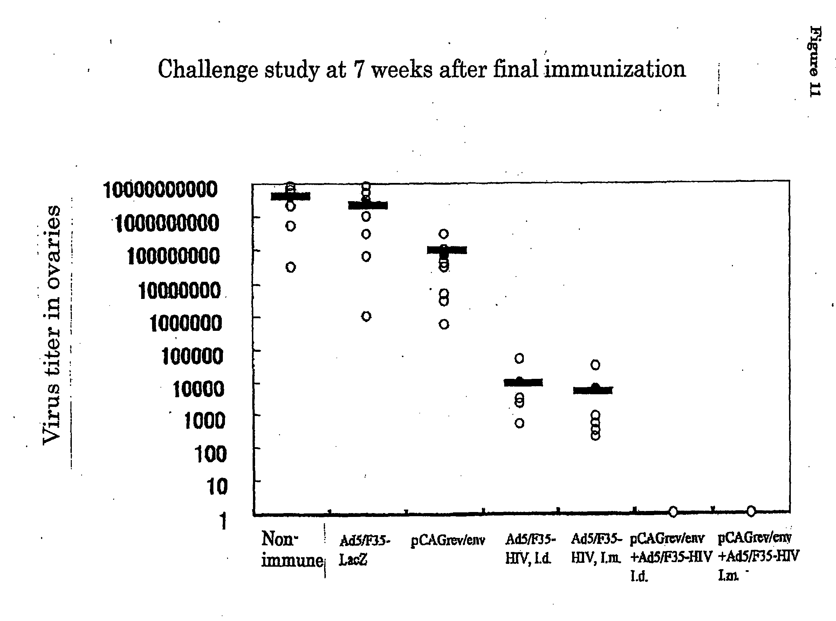

[Figure 11] Figure 11 shows immune response and the infection experiments results at 7 weeks after the final immunization. The mice were immunized with an i.m., or i.d. injection of DNA vaccine (pCAGrev/env) or virus vector (Ad5/F35-HIV) (10¹⁰ viral particles/mouse), either alone or in combination. Figure 11 shows the vaccinia virus vPE16 titer in mouse ovaries at 7 weeks after the final immunization.
   Each spot represents the virus titer of each mouse. A bar in the figure represents mean values of virus titers of 10 mice.

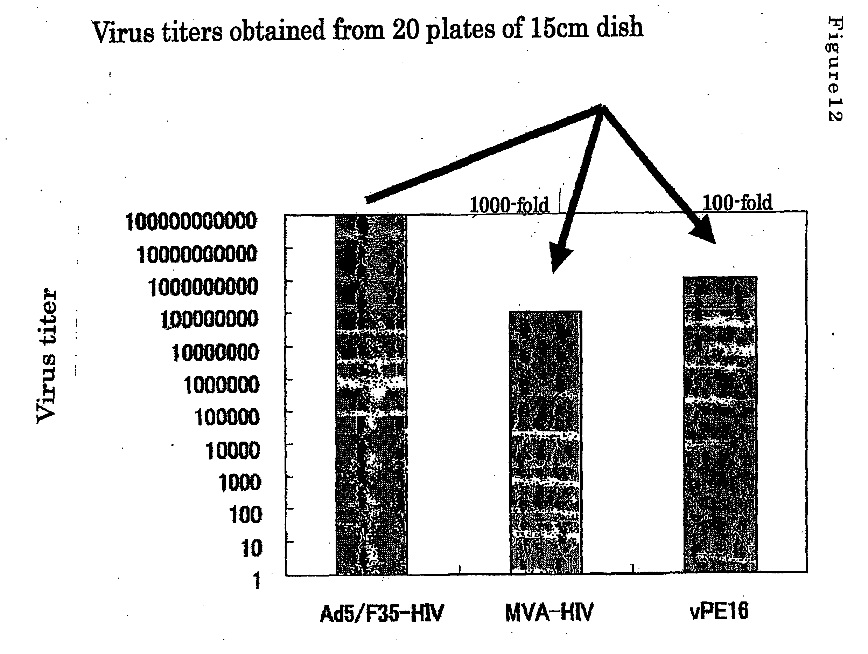

[Figure 12] Figure 12 shows titers of virus vectors (Ad5/F35-HIV, MVA-HIV and vPE16) obtained from 20 plates of 15cm dish culture.
[Figure 13] Figure 13 shows cell number of HIV-specific IFN-γ secreting spleen cells isolated from the mice immunized with Ad5/35-LacZ, Ad5/35-HIV clade C /env/gag of this inventionand from the mice with no-immunization.

## Claims

1. A chimera adenovirus type 5/type 11 or type 35 vector which comprises a replication-defective adenovirus type 5 and a gene coding for a human immunodeficiency virus (HIV) envelope protein or for a mutant thereof equivalent in function thereto as integrated in said adenovirus type 5 in a manner capable of expression, with the fiber protein-encoding gene of said adenovirus type 5 being substituted by a gene coding for the fiber protein of an adenovirus type 11 or type 35 or for a mutant thereof equivalent in function thereto in a manner capable of expression.

2. The chimera adenovirus type 5/type 11 or type 35 vector according to Claim 1, with a gene coding for a HIV clade B or HIV claid C envelope protein or for a mutant thereof equivalent in function thereto as integrated in a manner capable of expression.

3. The chimera adenovirus type 5/type 11 or type 35 vector according to Claim 1 or 2 which further comprises an HIV gag gene or a mutant gene thereof equivalent in function thereto as integrated therein in a manner capable of expression, together with said gene coding for HIV envelope protein or mutant thereof equivalent in function thereto.

4. The chimera adenovirus type 5/type 11 or type 35 vector according to any of Claims 1 to 3, with the fiber protein-encoding gene of said adenovirus type 5 being substituted by a gene coding for the fiber protein of an adenovirus type 35 or for a mutant thereof equivalent in function thereto in a manner capable of expression.

5. The chimera adenovirus type 5/type 11 or type 35 vector according to any of Claims 1 to 4, wherein the replication-defective adenovirus type 5 is an E1-deficient, replication-defective adenovirus type 5 or an E1 and E3-deficient replication-defect adenovirus type 5.

6. A pharmaceutical composition which comprises, as an active ingredient, the chimera adenovirus type 5/type 11 or type 35 vector according to any of claims 1 to 5.

7. The pharmaceutical composition according to Claim 6 which is to be used for the protection against HIV infection.

8. The pharmaceutical composition according to Claims 6 or 7 which is an HIV vaccine.

9. The pharmaceutical composition according to any of Claims 6 to 8 which is to be used in combination with a replication-defective virus vector or nonvirus vector comprising a gene coding for an HIV envelope protein or for a mutant thereof equivalent in function thereto as integrated therein in a manner capable of expression.

10. The pharmaceutical composition according to any of Claims 6 to 8 which is to be used in combination with an anti-HIV agent.

11. The pharmaceutical composition according to Claim 10, wherein the anti-HIV agent comprises at least one species selected from among reverse transcriptase inhibitors and protease inhibitors.

12. A method for the protection against HIV infection or vaccination against HIV which comprises administration of the chimera adenovirus type5/ type 11 or type 35 vector according to any of Claims 1 to 5 and a replication-defective virus vector or nonvirus vector comprising a gene coding for an HIV envelope protein or for a mutant thereof equivalent in function thereto as integrated therein in a manner capable of expression.

13. A method for the protection against HIV infection or vaccination against HIV which comprises administration of the chimera adenovirus type 5/type 11 or type 35 vector according to any of Claims 1 to 5 and an anti-HIV agent.
